# EUROPEAN PATENT SPECIFICATION

(11) **EP 1 210 440 B1**
(45) Date of publication and mention of the grant of the patent: **20.07.2005**
(21) Application number: 00955487.4
(22) Date of filing: 10.08.2000
(51) Int. Cl.: C12N 15/54, C12N 15/70, C12N 9/12, C12N 1/21, C12Q 1/68, C12P 19/34

(54) **TAQ DNA POLYMERASES HAVING AN AMINO ACID SUBSTITUTION AT E681 AND HOMOLOGS THEREOF EXHIBITING IMPROVED SALT TOLERANCE**
TAQ DNA-POLYMERASE MIT EINER AMINOSÄURESUBSTITUTION IN POSITION E681 UND ENTSPRECHENDE HOMOLOGE MIT EINER VERBESSERTEN SALZTOLERANZ, NÜTZLICH FÜR DIE SEQUENZIERUNG SCHWIERIGER TEMPLATESEQUENZEN MITTELS GELADENEN ODER UNGELADENEN TERMINATOREN
ADN POLYMERASES TAQ AVEC SUBSTITUTION D'ACIDE AMINE EN E681 ET LEURS HOMOLOGUES PRESENTANT UNE TOLERANCE ACCRUE AU SEL

(30) Priority: 21.08.1999 US 150167 P; 17.09.1999 US 154739 P
(43) Date of publication of application: 05.06.2002
(73) Proprietor: Amersham Biosciences Corp., Piscataway, N.J. 08855-1327 (US)
(72) Inventor: DAVIS, Maria, Piscataway, NJ 08855-1327 (US); NELSON, John, Piscataway, NJ 08855-1327 (US); KUMAR, Shiv, Piscataway, NJ 08855-1327 (US); FINN, Patrick, J., Piscataway, NJ 08855-1327 (US); NAMPALLI, Satyaam, Piscataway, NJ 08855-1327 (US); FLICKE, Parke, Piscataway, NJ 08855-1327 (US)
(74) Representative: Franks, Barry Gerard
(86) International application number: PCT/US2000/022150
(87) International publication number: WO 2001/014568

(56) References cited:
- EP-A- 0 655 506
- EP-A- 0 745 676
- EP-A- 0 902 035
- WO-A-98/40496
- US-A- 5 885 813

## Description

### CROSS-REFERENCE TO RELATED APPLICATIONS

This application claims priority under 35 USC § 119(e) of US provisional application serial number 60/150,167, filed on August 21, 1999, and US provisional application serial number 60/154,739, filed on September 17, 1999.

### BACKGROUND OF THE INVENTION

### Field of the Invention

The instant disclosure pertains to thermostable DNA polymerases which exhibit improved robustness and efficiency. In particular, the instant DNA polymerase has been shown to result in a substantial improvement of signal uniformity compared to Taq Δ271/F272M/F667Y DNA polymerase when used in DNA sequencing reactions.

### Background

DNA polymerases are enzymes which are useful in many recombinant DNA techniques such as nucleic acid amplification by the polymerase chain reaction ("PCR"), self-sustained sequence replication ("3SR"), and high temperature DNA sequencing. Thermostable polymerases are particularly useful. Because heat does not destroy the polymerase activity, there is no need to add additional polymerase after every denaturation step.

Naturally occurring DNA polymerases preferentially incorporate unlabeled nucleotides over corresponding labeled nucleotides into polynucleotides. This ability of DNA polymerases to discriminate against fluorescently labeled nucleotides had an undesirable effect on many molecular biology procedures that require the enzymatic addition of labeled nucleotides, e.g., labeled dideoxy terminator sequencing. Ambiguous sequencing determinations often result from the disproportionate number of labeled and unlabeled dideoxy terminators and nucleotides. On an electropherogram obtained from a capillary electrophoresis sequencing unit, this phenomena shows up as uneven peaks. Large signals due to a larger amount of incorporated labeled ddNTP (shown as wide peaks) can obscure smaller signals and lead to ambiguous sequence determinations. Additionally, many of the enzymes presently available are sensitive to high salt environments. '

Thus, a need continues to exist for an improved DNA polymerase having improved discrimination properties (and thus resulting in improved signal uniformity) and increased tolerance to high salt conditions. These and other concerns are addressed in greater detail below.

### BRIEF SUMMARY OF THE INVENTION

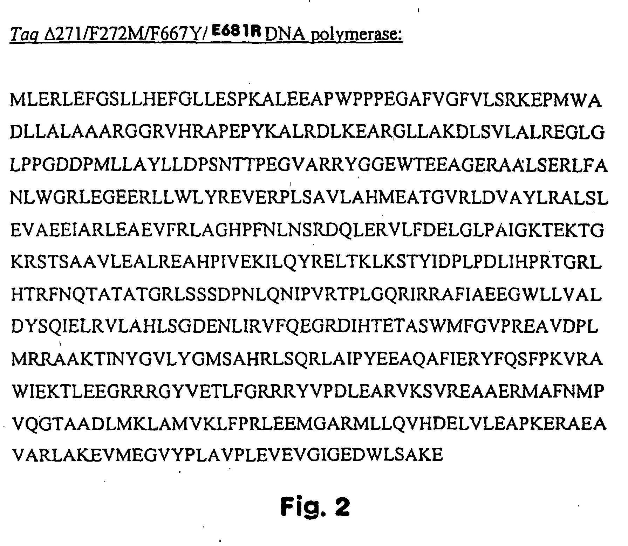

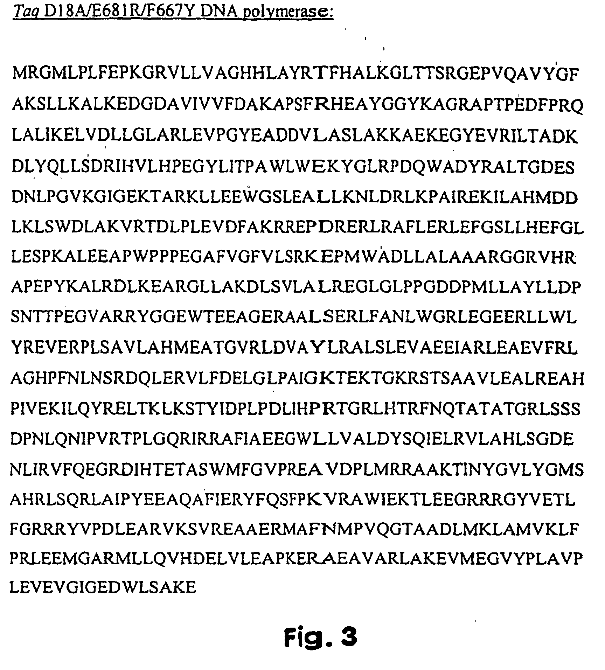

The instant disclosure teaches a purified recombinant thermostable DNA polymerase comprising the amino acid sequence set forth in Figure 2 or 3. The instant disclosure also teaches an isolated nucleic acid that encodes a thermostable DNA polymerase, wherein said nucleic acid consists of the nucleotide sequence set forth in Figure 2 or 3, as well as a recombinant DNA vector that comprises the nucleic acid, and a recombinant host cell transformed with the vector. The instant disclosure also teaches a method of sequencing DNA comprising the step of generating chain terminated fragments from the DNA template to be sequenced with the DNA polymerase in the presence of at least one chain terminating agent having a net negative or a net positive charge and one or more nucleotide triphosphates, and determining the sequence of said DNA from the sizes of said fragments. The instant disclosure also teaches a kit for sequencing DNA comprising the DNA polymerase and nucleic acid terminator having a net negative or a net positive charge.

### DETAILED DESCRIPTION

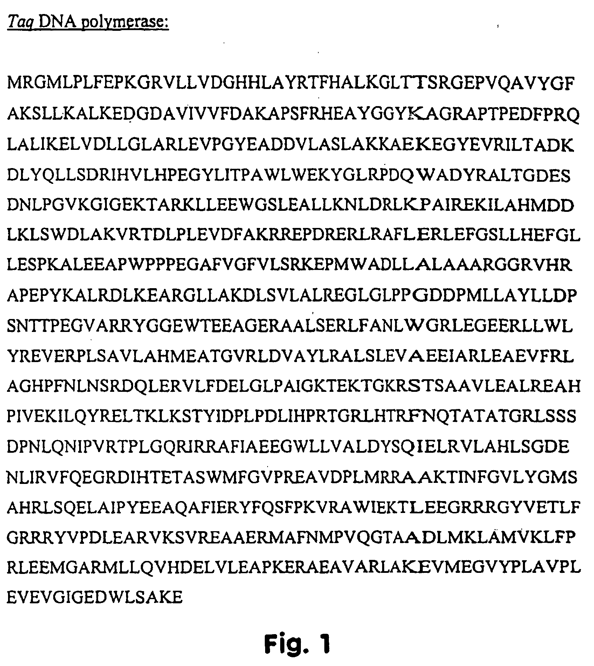

One objective of the instant disclosure is to increase the uniformity of dye-terminator incorporation in fluorescent dye DNA sequencing. One important DNA polymerase is Taq DNA polymerase isolated from the thermophilic bacterium *Thermus aquaticus*, the amino acid sequence for which is shown at Figure 1. The full length enzyme was truncated to eliminate 5' to 3' exonuclease activity and to provide a polypeptide more stable to proteolysis and heat treatment. The truncated enzyme is known as Taq Δ271/ F272M/F667Y DNA polymerase, which is commercially available from Amersham Pharmacia Biotech as Thermo Sequenase® DNA polymerase. Position 1 (amino acid Met) in Taq Δ271/ F272M/F667Y DNA polymerase corresponds to position 272 in full length Taq polymerase. It should be noted that the numbering used in the instant disclosure is that for Taq Δ271/ F272M/F667Y polymerase, not for Taq polymerase.

Single amino acid substitutions were introduced into Taq Δ271/ F272M/F667Y polymerase. These substitutions are designated as E344Q, I367V, F367Y, E416K and E410R. Each of the subsituted polymerases was expressed, purified, and analyzed for uniformity of dye-terminator incorporation in fluorescent sequencing studies, as assayed by signal uniformity. The E410R substitution was found to result in a substantial improvement of signal uniformity compared to Taq Δ271/ F272M/F667Y DNA polymerase.

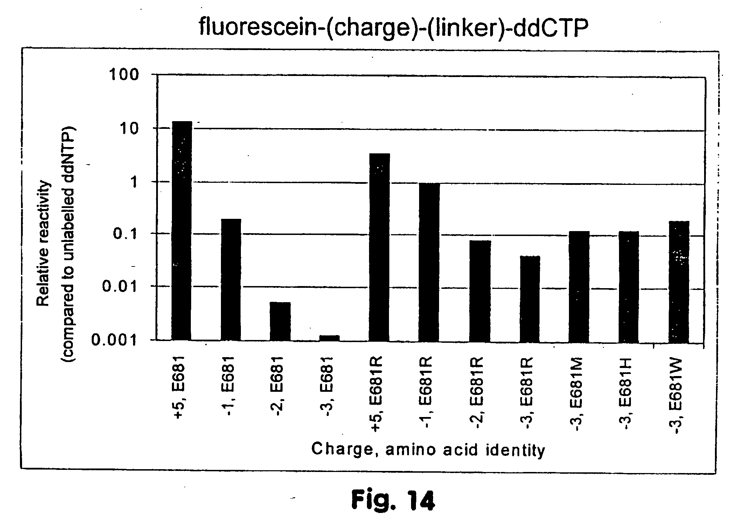

The DNA polymerases disclosed herein are especially suitable for use in sequencing reactions which employ terminators having a net positive or a net negative charge. Surprisingly, the instant DNA polymerases have been shown to modulate the incorporation of such terminators during the sequencing reaction. See for example Figure 14. Furthermore, such nucleic acid terminators, which along with the corresponding nucleic acid terminator decomposition products, migrate on separation media at different rates than the sequencing reaction products and which result in improved sequence data. These nucleic acid terminators also allow for the direct loading of nucleic acid sequencing reactions onto separating media. To achieve this goal, negatively or positively charged moieties are attached to the terminator molecule. The unreacted or degraded terminators containing such charged moieties move faster (negatively charged) or in the reverse direction (positively charged) than the DNA sequencing products.

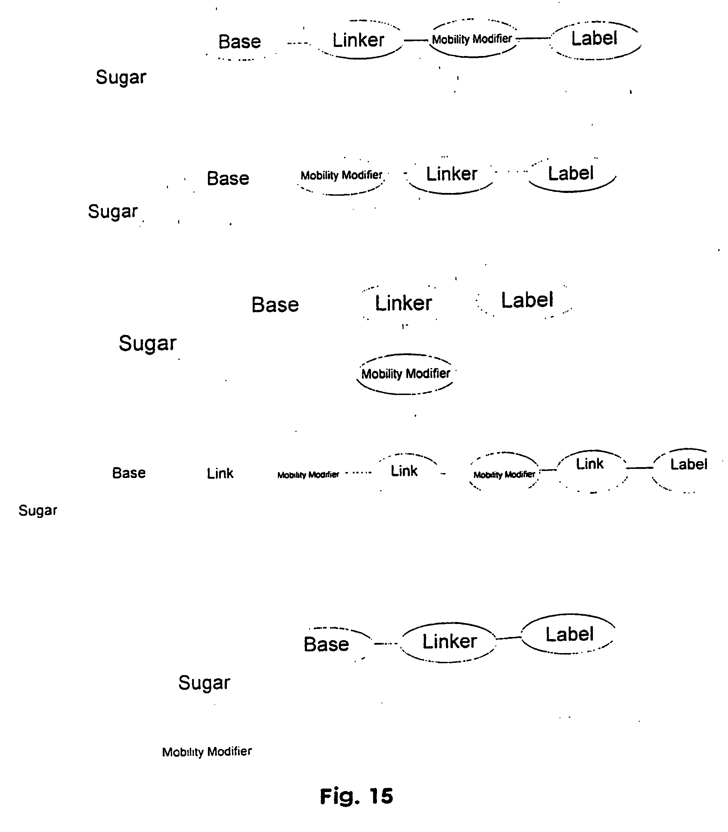

For example, the structures depicted in Figure 15 illustrate potential sites at which a charged moiety may be attached to a terminator. Referring to Figure 15, the Base may comprise A, T, G, C or analogs such as 7-deazapurine, inosine, universal bases. The Sugar may comprise furanose, hexose, mono-di-triphosphates, morpholine, didehydro, dideoxyribose, deoxyribose. The Linker may comprise 1-100 atoms, preferably 2-50 atoms consisting of C, H, N, O, S and halogens. The Mobility modifier may comprise any charged species which alters electrophoretic mobility of structure and degradation products, e.g., α-sulfo-β-alanine, cysteic acid, sulfonic acids, carboxylates, phosphates, phosphodiesters, phosphonates, amines, quaternised amines, and phosphonium moieties. The Mobility modifier may comprise a number of these units covalently linked together. The Label may comprise any signal moiety such as radioisotope, electrochemical tag, fluorescent tags, energy transfer (ET) labels, mass spectrometry tags, Raman tags, hapten, chemilluminescent group, enzyme, chromophore, and two or more labels. The label may also be charged, e.g. Cy5.5, bis-sulfonated carboxyfluorescein, or a dye attached to a charged moiety, e.g., carboxyfluorescein attached to cysteic acid or similar charged species. Methods for making these and other compounds are disclosed in U.S. Provisional Application No. 60/098,469 filed on August 31, 1998, and U.S. Application No. 90/018,695 filed on February 4, 1998, and PCT/GB98/00978 filed on April 2, 1998 and published on October 8, 1998, the disclosures of each application are incorporated in their entirety by reference herein.

The following examples are for illustration purposes only and should not be used in any way to limit the appended claims.

### EXAMPLES

### EXAMPLE 1

The construction, expression and purification of Taq Δ271/F272M/F667Y/E410R polymerase is described below. The other substitutions named above were constructed, expressed and purified in a similar manner.

### Construction

Primers BamHIFOR (5' ccg ctt ggg cag agg atc cgc cgg gcc ttc atc gcc gag ga) and NheIREV (5' tcg taa ggg atg gct agc cgc tgg gag agg cgg tgg gcc gac) were used in a standard PCR reaction to amplify the region between the BamHI and NheI restriction sites in pREFY2pref (cloned Taq Δ271/F272M/F667Y DNA polymerase). Primer BamHIFOR contains a BamHI restriction site which corresponds to the same unique site in pREFY2pref, and primer NheIREV contains a NheI restriction site which corresponds to the same unique site in pREFY2pref. In addition, primer NheIREV was designed to change the codon at position 410 from gag (encoding amino acid E, glutamic acid) to cgg (amino acid R, arginine). The PCR product was digested with the appropriate enzymes, and isolated by agarose gel electrophoresis. The large fragment resulting from the BamHI/NheI digestion of pREFY2pref was also gel purified, and ligated to the PCR fragment above. Following transformation into E. coli, plasmid DNA was isolated and subsequently sequenced to confirm the presence of the E410R substitution. The amino acid sequence for Taq Δ271/F272M/F667Y/E410R DNA polymerase is shown at Figure 2.

### Expression & Purification of the Tag Δ271/F272M/F667Y/E410R Polymerase

Vector pRE2 which carries the lambda p_{L} promoter was used with an *E*. *coli* strain which has the heat labile repressor protein cI857 to express the Taq Δ271/F272M/F667Y/E410R polymerase. This combination permits cultivation at 30°C followed by expression of a plasmid-borne protein at elevated temperatures such as 42°C. Liquid cultures were typically grown at 30°C to an OD₆₀₀ of ~ 1.0, and then transferred to 42°C for ~ 2.5 hours. Bacterial cells were harvested by centrifugation, resuspended in lysis buffer (50 mM Tris-HCl pH 8.5, 1 mM EDTA, 0.1% Tween-20, 0.1% Triton X-100, 10 mM MgCl₂, and 16 mM (NH₄)₂SO₄), and then heated at 80°C for 20 minutes to precipitate *E. coli* proteins. The heat lysate was clarified by centrifugation, and supplemented with 300 mM NaCl, and applied to a DE52 anion exchange column (commercially available from Whatman). The flow-through was diluted in Buffer A (50 mM Tris-HCl pH 8.0, 1 mM EDTA, 10% glycerol, 0.1% Triton X-100 and 0.1% Tween-20) to reduce the NaCl concentration to 100 mM, and applied to a Heparin Sepharose column (commercially available from Pharmacia Inc.). The column was developed by linear gradient from 100 to 700 mM NaCl in Buffer A. The enzyme eluted at ~250mM NaCl. Fractions containing polymerase activity were pooled, concentrated on a Centriprep-50 apparatus (commercially available from Amicon) and dialyzed extensively against a final buffer containing 20 mM Tris-HCl pH 8.5, 50% glycerol, 0.1 mM EDTA, 0.5% Tween-20, 0.5% Triton X-100, 100 mM KCl and 1 mM DTT. The purity of the polymerase preparation was confirmed by SDS-PAGE.

### Enzyme Characterization

### 1) Salt tolerance:

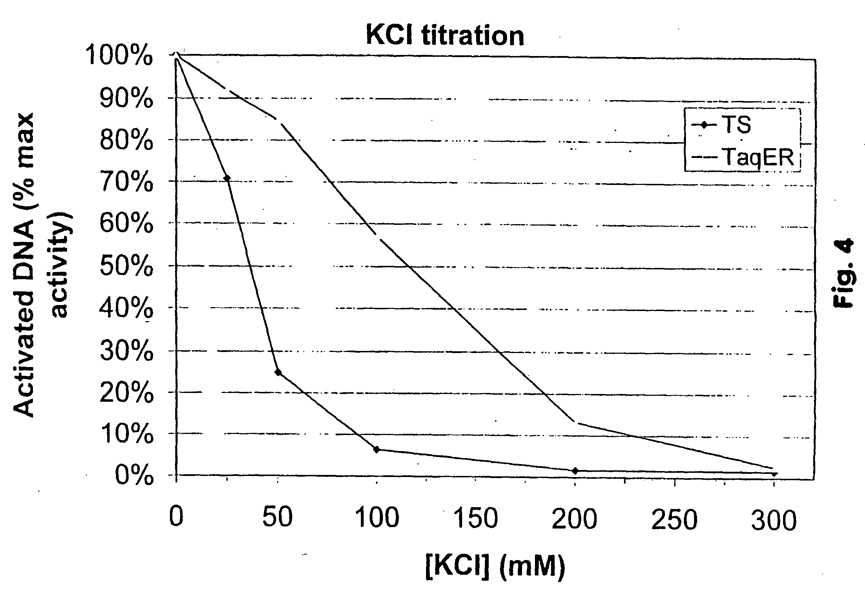

The Taq Δ271/F272M/F667Y/E410R DNA polymerase activity has been examined under a KCl titration experiment by using both activated salmon sperm DNA and primed M13 DNA as substrates. In both assays, Taq Δ271/F272M/F667Y E410R showed a decreased polymerase activity while increasing KCl concentration from 0 to 200 mM. However, the enzyme displays a much slower activity decrease compared to TS. Figure 4 plots the data from KCl titration of Taq Δ271/F272M/F667Y and Taq Δ271/F272M/F667Y/E410R using activated salmon sperm DNA as substrates. The 50% KCl inhibition for Taq Δ271/F272M/F667Y/E410R polymerase activity with activated salmon sperm or primed M13 DNA are 120 mM and 100 mM, respectively compared to TS, which has a 50% KCl inhibition of 35 mM. The polymerase assay buffer contains: 25 mM TAPS (pH 9.3), 2 mM MgCl₂, 1 mM 2-inercaptoethanol and 200 mM each dNTP plus 0.05 Ci/mmol [α-³³P]-dATP. A comparison of salt tolerance data for Taq Δ271/F272M/F667Y and substitutions thereof is presented below in Table I.

**TABLE I**

| Enzyme, substitution | Salt Tolerance |
|---|---|
| Taq Δ271/F272M/F667Y | 35 mM |
| Taq Δ271/F272M/F667Y/E410R | 135 mM |
| Taq Δ271/F272M/F667Y/E410M | 125 mM |
| Taq Δ271/F272M/F667Y/E410W | 125 mM |
| Taq Δ271/F272M/F667Y/E410H | 110 mM |

### 2) Thermostability at 95°C:

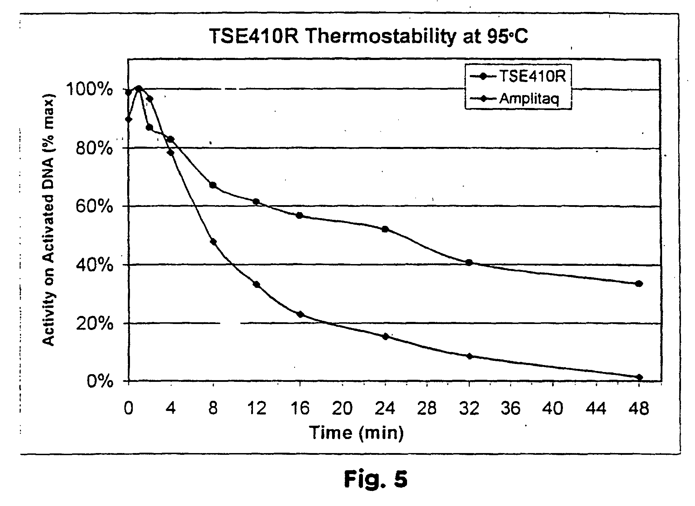

The thermostability of Taq Δ271/F272M/F667Y/E410R has been assayed as follows. First, the 95°C heating step was performed in a buffer containing 50 mM Tris-HCl pH 9.5, 5mM MgCl₂, 50µM each dNTP and 100ng M 13 single strand DNA. Then 10 units of enzyme were mixed with the above solution and a time course performed by taking aliquots (20 µl each) and placing on ice. Next, dilutions were made in a buffer containing 10 mM Tris-HCl, pH 8.0, 1 mM 2-mercaptoethanal, 0.5% Tween-20, 0.5% Nonidet P-40. In the third step, the heated and diluted samples have been assayed for survivor polymerase activity under a standard polymerase assay condition described in section (1) but including 50 mM KCl. Figure 5 showed the thermostability assay of comparing Taq Δ271/F272M/F667Y/E410R with Amplitaq. The 50% inhibition time at 95°C for Taq Δ271/F272M/F667Y/E410R and Amplitaq are 25 min and 8min, respectively.

### 3) Processivity assay:

The processivity of Taq Δ271/F272M/F667Y/E410R has been examined in an enzyme dilution method, which insures that the polymerase activity is assayed for a single enzyme binding event. The assay buffer contains 15 mM Tris-HCl (pH 9.5), 3.5 mM MgCl₂, 100 mM each dNTP and 1µg P³³ labeled primed M13. The primer extension experiment has been performed at 65°C for 90 seconds. The samples were analyzed on a 8% polyacrylamide-7 M urea sequencing gel. Taq Δ271/F272M/F667Y/E410R has an increased processivity of about 30 nucleotides per polymerase binding event. This is about a 7 to 8 fold increases compared to Taq Δ271/F272M/F667Y (4 nt/binding event).

### 4) Uniform termination events:

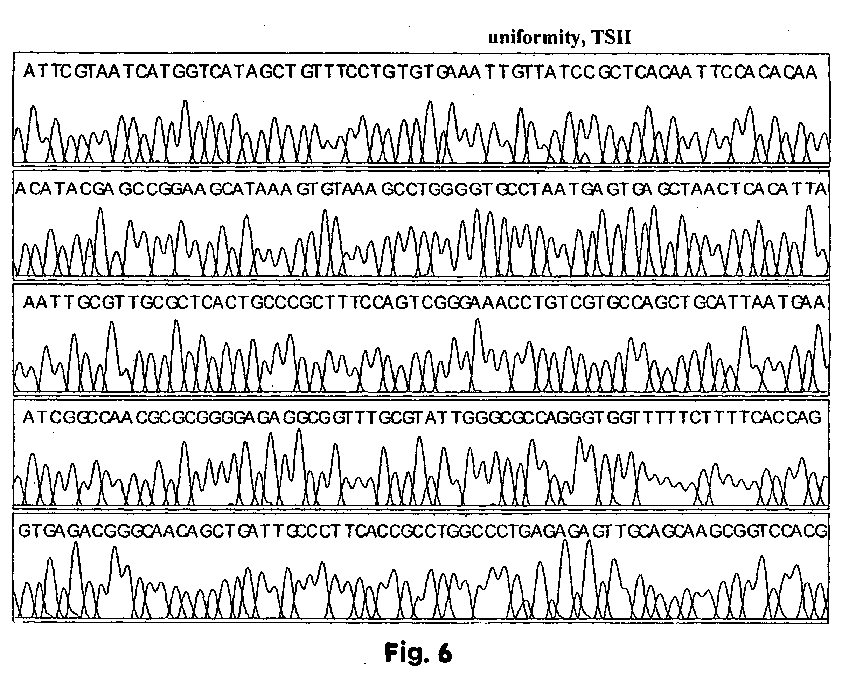

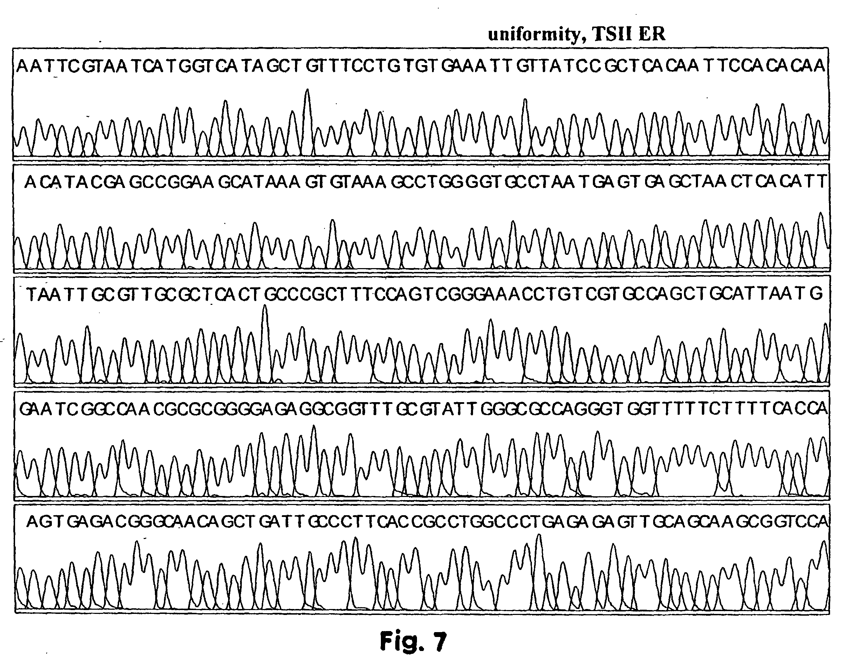

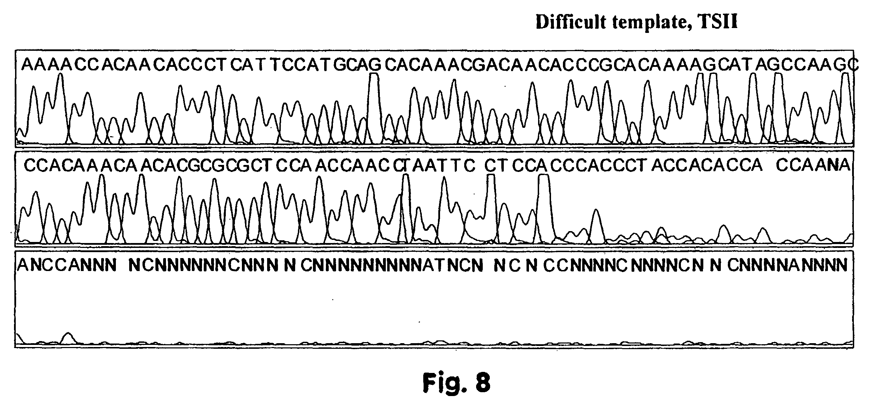

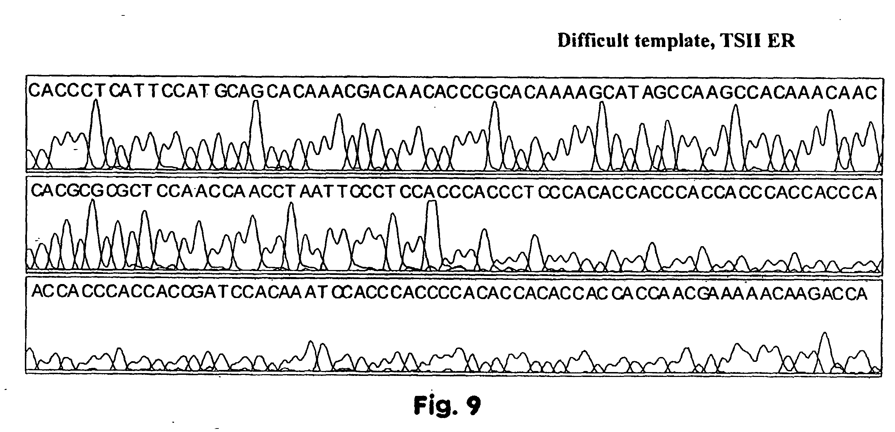

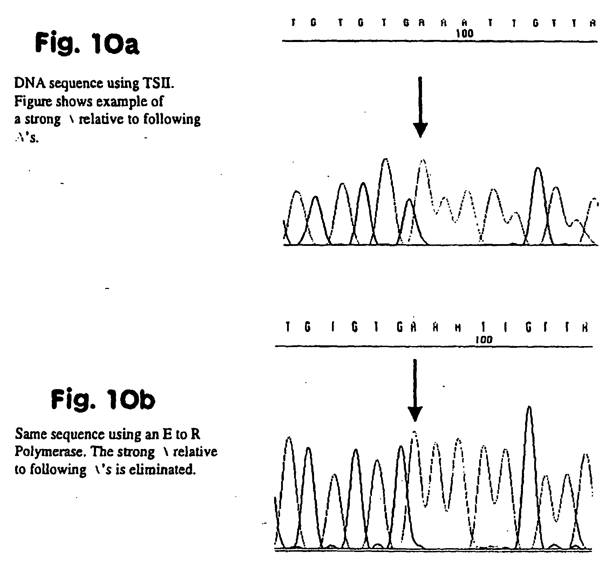

The new E to R amino acid modification discovered also results in increased uniformity in termination events during sequencing reactions containing net positive, negative, or neutrally charged dideoxynucleotide terminators. This results in an increased uniformity in electropherogram band intensity and an increase in the number of bases which can be basedcalled per sequence. For example, as shown in Figure 6, the average deviation of band intensity using Thermosequenase Version II is about a 30% deviation. However, as shown in Figure 7, a typical result using an E to R polymerase is about a 22% deviation. This improvement is significant. Portions of Figures 6 and 7 are magnified in Figures 8 through 10 for comparison purposes.

### 5) Ability to sequence difficult areas:

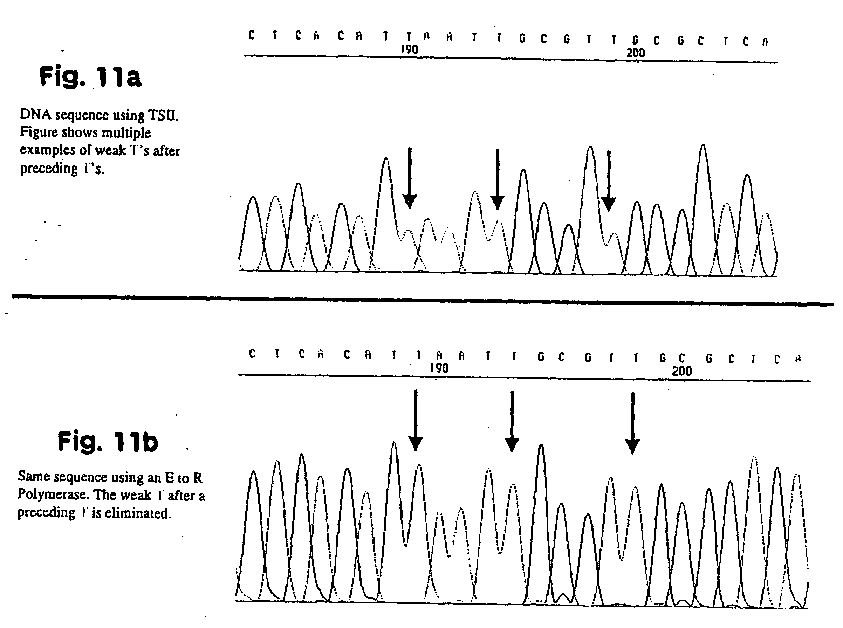

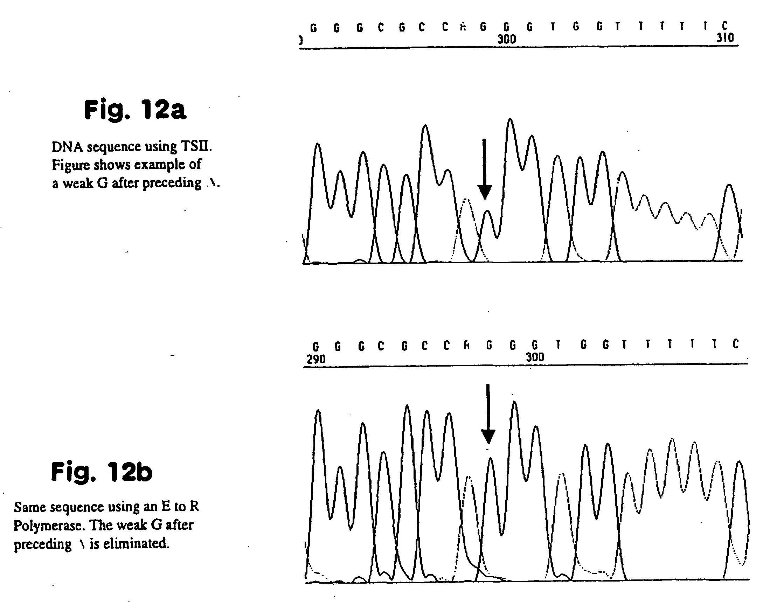

The new E to R amino acid modification discovered also results in an improved ability to sequence DNA's which contain "difficult to sequence" areas. Certain specific DNA sequences are extremely likely to cause sequencing DNA polymerases problems, resulting in a reduced quality of the sequence obtained (see Figure 11). Surprisingly, enzymes containing the E to R modification are much more likely to yield higher quality sequence data from DNA containing these difficult to sequence areas (see Figure 12).

### EXAMPLE 2: TAQ D18A/E681R/F667Y POLYMERASE

We also constructed using standard techniques described above a full length version of Taq polymerase with the following substitutions : D18A/E681R/F667Y. In this enzyme, the D18A substitution removes the 5' to 3' exonuclease activity, rather than the deletion of amino acids as in the Taq Δ271/F272M/F667Y DNA polymerase polypeptide. The E681R substitution is the position equivalent to E410R in Taq Δ271/F272M/F667Y DNA polymerase, and F667Y is the equivalent position to F396Y in Taq Δ271/F272M/F667Y DNA polymerase. This enzyme also has properties desirable for sequencing with dye terminators. The amino acid sequence of Taq D18A/E681R/F667Y DNA polymerase is shown at Figure 3.

Uniformity of positive terminator reactions is improved considerably with the substitutions at E681 as shown by the data in Table II below.

**TABLE II**

| Enzyme, substitution | Uniformity (r.m.s.) |
|---|---|
| TSI, E681 | 0.52 |
| TSI, E681R | 0.39 |
| TSI, E681H | 0.37 |
| TSI, E681I | 0.4 |
| TSI, E681M | 0.31 |
| TSI, E681 W | 0.34 |

Root mean square ("r.m.s.") is a measure of uniformity of a four color sequence reaction. This experiment used positive terminators (5 lysines in the linker) and standard sequencing reaction conditions. The improvement of 0.52 to below 0.45 shows a significant increase in uniformity for the sequencing reaction.

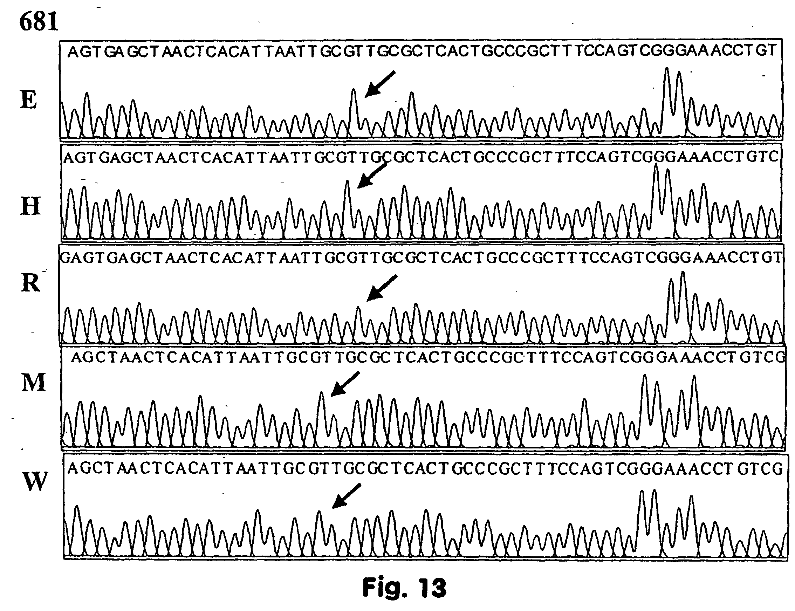

Figure 13 is a side-by-side comparison of electropherograms obtained from four color sequencing reactions conducted using D18A/F667Y DNA polymerases having various E681 substitutions as described at the left of each electropherogram. As shown in Figure 13, D18A/E681R/F667Y shows the most uniform peak heights and thus the most improvement in uniformity.

Figure 14 shows the relative reactivity compared to unlabelled ddNTPs evidenced in four color sequencing reactions which employed D18A/F667Y and various E681 substitutions therEof with various charged terminators.

### Nucleic Acid Terminators

### 1. An example of charge modified reporters as applied to direct load

### 1.1 Chemistry

The following scheme was used to synthesize labeled ddNTPs with a charged reporter moiety. The linker was synthesized according to methods disclosed in U.S. Provisional Application No. 60/098,469 filed on August 31, 1998, the entire disclosure of which is hereby incorporated by reference herein.

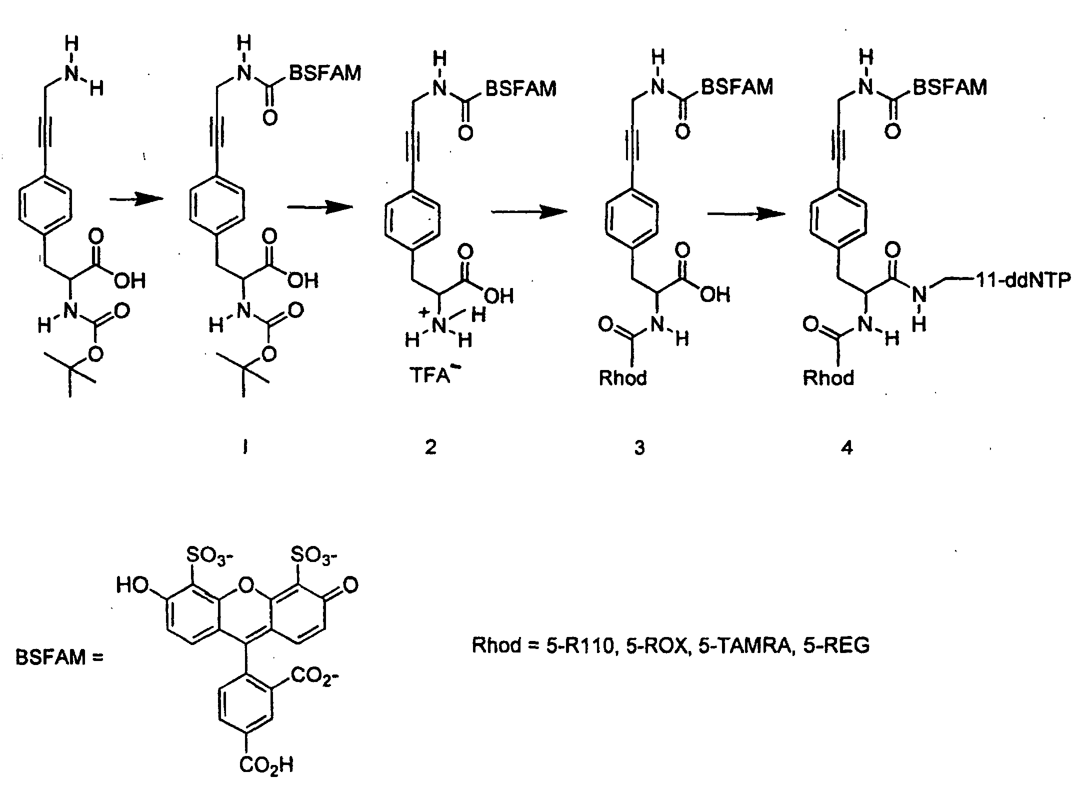

### 1.2 Discussion

4',5' Bis-sulfono-5-carboxyfluorescein (BSFAM) was attached to 4-propargylamino-N-α-*t*-butoxycarbonylphenylalanine by initial formation of the corresponding N-hydroxysuccinimide active ester using TSTU in DMF/diisopropylethylamine. Activation times were typically 15 minutes as observed by tlc before addition of the amino component. The product 1 was isolated by C18 RP-HPLC then treated with neat trifluoroacetic acid to remove the carbamate moiety, with the product 2 isolated by Et₂O precipitation. Attachment of the rhodamine moiety was carried out using 5-rhodamine hydroxysuccinimde active esters in DMSO/diisopropylethylamine. All the double dye cassettes were purified by reverse phase HPLC prior to conjugation to alkylamino ddNTPs using published methods (and as disclosed in U.S. Provisional Application No. 60/098,469 filed on August 31, 1998, the entire disclosure of which is hereby incorporated by reference herein). The labeled ddNTPs were purified by silica gel chromatography followed by ion exchange chromatography then reverse phase HPLC.

### 1.3 Experimental

All chemicals were purchased from Sigma, Aldrich, Fluka or Fisher Scientific unless stated otherwise. UV/visible spectra were recorded on a Perkin Elmer Lambda 20 UV/visible spectrophotometer in conjunction with Winlab™ software.

### 4-(propargylamido-4',5'-bissulfonatefluorescein)-N-α-t-butoxycarbonylphenylalanine (1)

4'-5'-bissulfono-5-carboxyfluorescein (100mg, 0.18mmol) was dissolved in DMF (4ml) then diisopropylethylamine (0.48ml, 15 eq.) and TSTU (65mg, 1.2eq.) added. The reaction mixture was stirred at room temperature for 1h. then 4-propargylamino-N-α-*t*-butoxycarbonylphenylalanine (69mg, 1.0eq) added. Stirring was continued for 3h. then the reaction mixture evaporated to dryness *in vacuo*. The product was isolated by reverse phase HPLC (C18, DeltaPak 15µ, 100A, 50x300µm) eluting with 0-100% eluant B over 60 min (A = 0.1M TEAB, B = 50% MeCN/0.1MTEAB v/v, 100ml/min.). The product (retention time 37 min.) was evaporated to dryness *in vacuo* then coevaporated with MeOH (3x10ml) before lyophilization (100mg, 65%). UV/vis (1M triethylammonium bicarbonate pH 8.8) 495nm (24670), 465nm (shoulder, 9634), 312nm (6708).

### 4-(propargylamido-4',5'-bissulfonatefluorescein)-phenylalanine-α-ammonium trifluoroacetate (2)

4-(propargylamido-4',5'-bissulfonatefluorescein)-N-α-*t*-butoxycarbonylphenylalanine (100mg, 0.12mmol) was treated with trifluoroacetic acid (10ml) for 15min. then evaporated to dryness *in vacuo*. The residue was coevaporated with toluene (3x10ml) then the product precipitated by the addition of Et₂O (50ml). The solid formed was collected by filtration, washed with cold Et₂O (3x50ml) then dried under high vacuum (100mg, 99%). Rf (tlc, iPrOH:NH₄OH:H₂O (6:3:1)=0.

### General methodology for the attachment of rhodamine dyes to 2 (3)

4-(propargylamido-4',5'-bissulfonatefluorescein)-phenylalanine-α-ammonium trifluoroacetate **2** (0.1 mmol) was dissolved in DMSO (1ml) then diisopropylethylamine (0.26ml, 15 eq.) and rhodamine-NHS active ester (1.5 eq.) added. The reaction mixture was stirred at room temperature for 16h, then evaporated to dryness *in vacuo*. The R110 analog was treated with triethyammonium bicarbonate solution (0.1M, 50ml) for 16h to remove the trifluoroacetimido protecting groups then the product purified by RP-HPLC using identical conditions to **1** unless stated. Retention times (BSFAM/R110 = 31min, BSFAM/R110 = 55min 0-100% B over 90 min, 100 ml/min., BSFAM/REG 54min 0-100%B over 90 min., 100ml/min, BSFAM/TAMRA = 52min 0-100% B over 90 min). All absorption spectra show the presence of both dyes.

### General Methodology for Attachment of 3 to alkylamino-2',3'-dideoxynucleotide triphosphates (4).

The double dye cassette (1mmol) was dissolved in DMF (5ml) then disuccinimdyl carbonate (4eq.) and DMAP (4eq.) were added at -60°C. The reaction mixture was stirred at -30°C for 15 min. then a solution of aminoalkyl-ddNTP (0.67eq., Na₂CO₃/NaHCO₃ pH 8.5) added.

The reaction was stirred at room temperature for 1h. then applied directly to a SiO₂ gel column. The product was eluted with iPrOH:NH₄OH:H₂O (4:5:1 v:v:v) then evaporated to dryness *in vacuo* before subsequent purification by ion exchange chromatography then C 18 reverse phase HPLC as for **1**. Absorption spectra of each compound showed the presence of both dyes.

### 1.4 Comparative Electropherograms

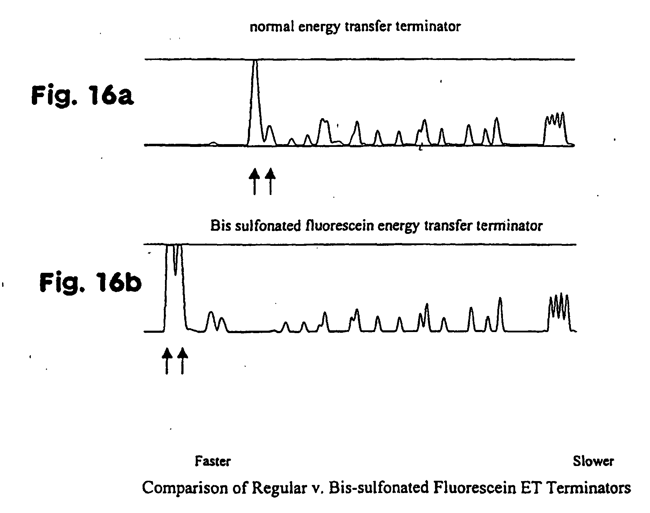

One of the terminators (structure 4, Rhodamine =5-ROX and N = C) formed above was used in a sequencing reaction and run on a slab gel. The resulting electropherogram is shown in Figure 16 which provides an example of the increase in migration rate relative to sequence products of unincorporated bis-sulfonated fluorescein energy transfer terminators (and thermal breakdown products thereof) compared to the migration rate of the regular ET terminators.

### 2. An example of a negatively charged linker arm as applied to direct load

### 2.1 Background

By incorporation of a number of charged amino acids onto a fluorescent reporter, it is possible to synthesize a labeled ddNTP containing extra negative charge that alters the mobility of the degradative by-products observed in a sequencing reaction.

### 2.2 CHEMISTRY

In order to determine the amount of negative charge required to remove colored by-products from the sequence ladder, fluorescein was attached to α-sulfo-β-alanine to form **5**. Compound **5** was attached to a 11-ddCTP (11=number of atoms in linker arm) to form **7**. A portion of **5** was attached to a second α-sulfo-β-alanine moiety to form **6** which was subsequently attached to 11-ddCTP to form **8.** A control ddNTP containing regular FAM attached to 11-ddCTP was also synthesized. The structures were run in a single color sequencing reaction to determine the effect of the charge on mobility.

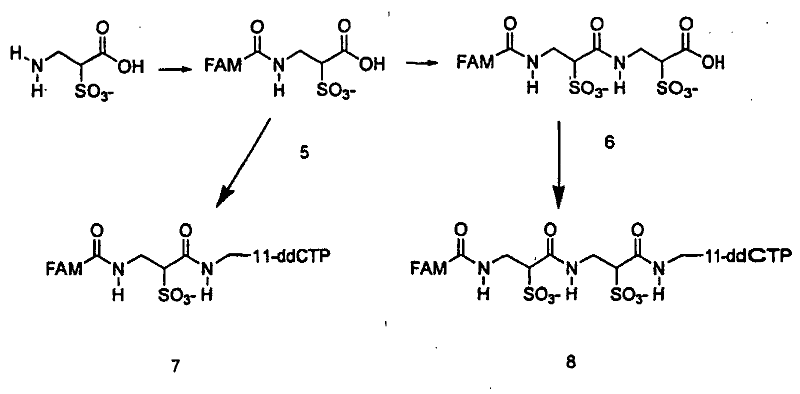

As fluorescein carries a net 1- charge, compound 7 is considered as overall 2- linker arm, compound 8 has an overall 3- linker arm charge.

### 2.3 Experimental

### N-5-carboxamidofluorescein-α-sulfo-β-alanine (5)

α-sulfo-β-alanine (59mg, 0.35mmol) was dissolved in DMF (2ml) then diisopropylethylamine (0.9mol, 15eq) added followed by S-FAM-NHS active ester (200mg, 1.2eq.). The reaction mixture was stirred at room temperature for 3h. then evaporated to dryness *in vacuo.* The residue was coevaporated with MeOH (10ml) then the product isolated by C18 RP HPLC (A=0.1MTEAB, B=0.1MTEAB, 50%MeCN v/v) eluting with 0-100%B over 90 min at 100ml/min. ¹H nmr (300MHz, CD₃OD); 1.27(t, 24H, J=8.4Hz, NCH₂CH₃), 3.05(q, 16H, J=8.4Hz, NCH₂CH₃), 3.95-4.05(m, 3H, CH₂+CHSO₃), 6.58(m, 3H, Ar-H), 6.85(d, 2H, J=11.0Hz, Ar-H), 7.30(d, 2H, J=11.0Hz, Ar-H), 8.02(d, 1H, J=7.6Hz, ArH), 8.45(s,1H,Ar-H).

### N-(N-5-carboxamidofluorescein-α-sulfo-β-alanine)amido-α-sulfo-β-alanine (6)

N-5-carboxamidofluorescein-α-sulfo-β-alanine (5, 50mg, 0.095mmol) was dissolved in DMF (3ml) then diisopropylethylamine (0.25ml, 15eq.) and TSTU (42mg, 1.5eq.) added. The reaction mixture was stirred at room temperature for 1h. then α-sulfo-β-alanine (24mg, 1.5eq.) added. Stirring was continued for 3h. then the reaction evaporated to dryness *in vacuo*. The product was isolated by ion exchange chromatography (mono-Q column, A=0.1M TEAB, 40%MeCN v/v, B=1.0M TEAB, 40%MeCN v/v, 0-50%B over 22min., 50-75%B from 22-50min. 75-100%B from 50-70 min., 4ml/min., retention time = 75-80min.) then C18 RP HPLC (A=0.1M TEABB=0.1M TEAB/MeCN 50% v/v, 0-100%B over 90 min., 100ml/min, retention time = 33min.). Rfᵢ(PrOH6:ammonia3:water1v/v/v) 0.34.

### General Methodology for Attachment of modified dyes to alkylamino-2',3'-dideoxynucleotide triphosphates (7,8).

The modified dye (1mmol) was dissolved in DMF (5ml) then disuccinimdyl carbonate (4eq.) and DMAP (4eq.) were added at -60°C. The reaction mixture was stirred at -30°C for 15 min. then a solution of aminoalkyl-ddNTP (0.67eq., Na₂CO₃/NaHCO₃ pH 8.5) added. The reaction was stirred at room temperature for 1h. then applied directly to a SiO₂ gel column. The product was eluted with iPrOH:NH₄OH:H₂O (4:5:1 v:v:v) then evaporated to dryness *in vacuo* before subsequent purification by ion exchange chromatography then C18 reverse phase HPLC as for 1.

### 2.4 Results

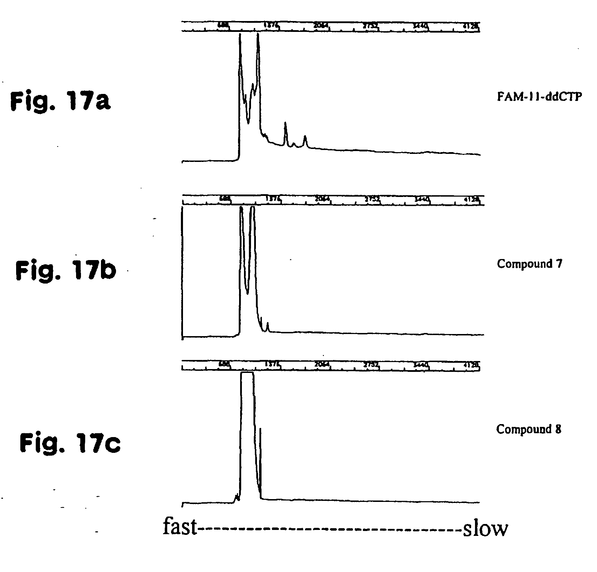

Each labeled ddNTP was dissolved in sequencing buffer and subjected to several rounds of thermocycling. The products were separated on a sequencing gel and the electropherograms shown in Figure 17. Interpretation of the electropherogram provided the conclusion an overall 3- charge (i.e., structure 8) removed the colored by-products from the area of the electropherogram where true sequencing data would be obtained.

Figure 17 illustrates how the net negative charge of the dye labeled dideoxynucleotides affects their (and thermal breakdown products thereof) migration rate. As the net negative charge of the terminator increases, the migration rates of the various peaks seen (each of the peaks seen are either dye labeled dideoxynucleotides or thermal breakdown products thereof) increases (Figure 17). At an overall 3- charge (2- from linker, 1-from fluorescein) peaks are absent from the region of the electropherogram where true sequence data would normally be obtained.

### 3. Negatively charged extended linker arms as applied to direct load

### 3.1 Background

In order to improve the efficiency of incorporation of the modified terminator, a labeled terminator with a 3- charge on the linker arm was synthesized, this time containing an extended linker arm of 18 and 24 atoms.

### 3.2 Chemistry

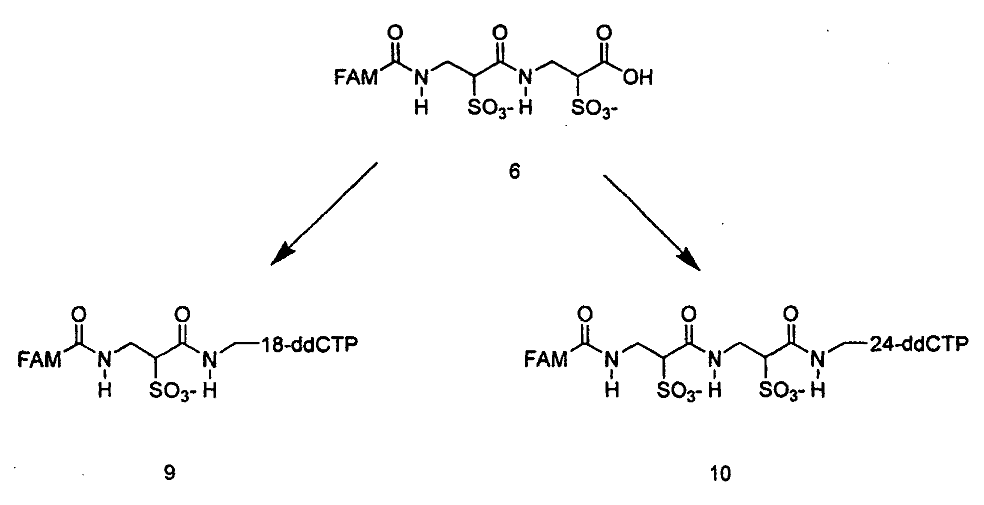

### 3.3 Experimental

Compound 6, was attached to 18-ddCTP and 24-ddCTP using the standard protocol for attachment of labels to ddNTPs outlined in section 2.3. The method of purification was the same for 9 and 10.
- Retention time of 9:: Mono-Q™ ion exchange (47min)
- Retention time of 10:: Mono-Q™ ion exchange (42min)
C18 RP-HPLC (15min)

### 3.4 Sequencing Results

From the sequencing experiments it was clear that increasing the linker arm length improved incorporation of the terminator. This information, coupled to the presence of the 3- charge in the dye-linker structure led us to investigate rhodamine dyes with a 3- charged linker. This would permit four color sequencing.

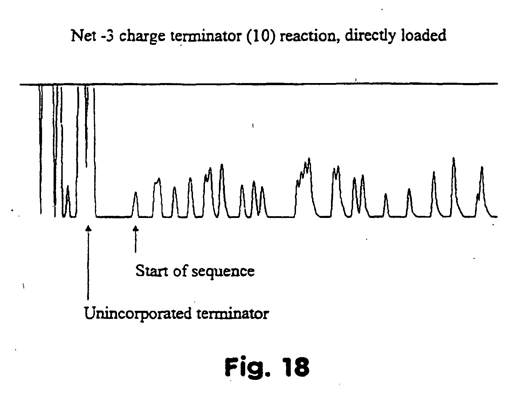

As shown in Figure 18, it is possible to directly load a sequencing reaction with no clean-up procedure. Figure 18 shows no peaks resulting from unincorporated dye-labeled terminator in the sequence, thus demonstrating the utility of negatively charged terminators with respect to direct load sequencing.

### 3.5 Rhodamine Labeled Terminators Containing a 3- Linker Arm

The following chemistry was attempted to synthesize a set of four differently labeled terminators:

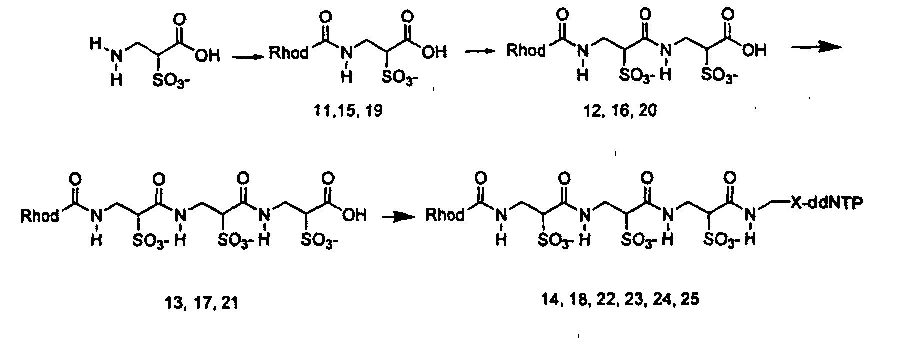

**TABLE III**

| Compound Nos. | Rhod | X | N |
|---|---|---|---|
| 11-14 | REG | 24 | U |
| 15-18 | TAMRA | 24 | A |
| 19-22 | ROX | 24 | G |
| 23 | TAMRA | 12 | A |
| 24 | ROX | 12 | G |
| 25 | ROX | 18 | G |
| Rhod = rhodamine label, X = length of linker arm, N=base | | | |

### 3.6 Experimental

Compounds 11,15,19 were synthesized according to the method outlined for 5.
Compounds 12, 13,16,17,17,21 according to the method outlined for 6.
Compounds 14, 18,22-25 according to the general methodology for attachment of modified dyes to alkylamino-2',3'-dideoxynucleotide triphosphates (7,8).

### 3.7 Results and Discussion

The labeled triphosphates 14, 18, 22 were used in a direct load sequencing experiment. Compound 14 in a direct load experiment showed no breakdown products and with TSII and TaqERDAFY. Compounds 18 and 22 gave very dark sequencing bands and were observed to be forming an unexpected aggregate (as observed in the emission spectrum). The compounds also produced large colored blobs on a sequencing gel which interfered with interpretation of the sequence.

In order to overcome the aggregation effect, structures 23-25 were synthesized to investigate the effect of a shorter linker arm. Compound 23 has been shown to yield a clean sequence, 24 and 25 are awaiting testing. Structures 23-25 all have the expected rhodamine emission spectrum hence it appears that the aggregation problem may have been overcome.

### 4 Other examples of negatively charged linker arms

Other negatively charged linker arms have been synthesized and studied for example the phopshodiester structure shown below. The product was synthesized using phopshoramidite chemistry however it could also be synthesized via H-phosphonates, phosphoroimidazolides, or phosphotriester chemistry.

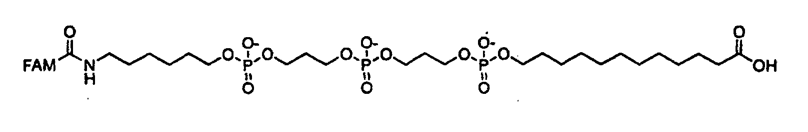

### 5. Examples of Tenninators with Positively Charged Reporters

### 5.1 Background

In order to study positively charged structures, the following labeled terminator was synthesized.

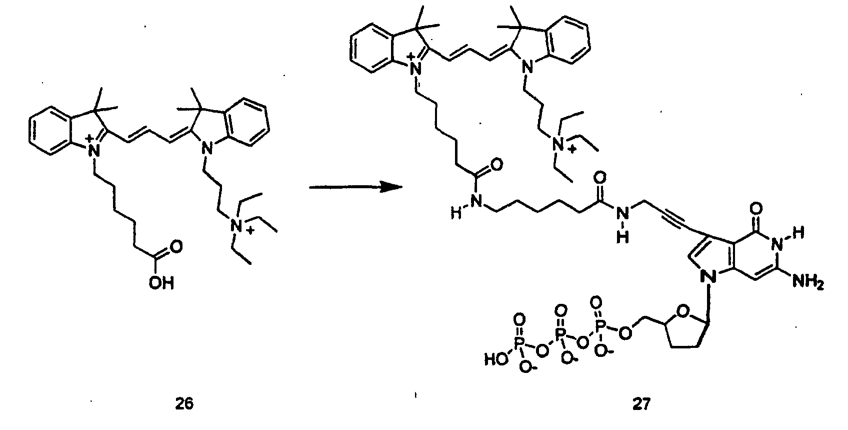

### 5.2 Experimental

Compound 26 (10mg, 0.0134mmol) was dissolved in DMF (1ml) then diisopropylethylamine (23µl, 10eq.) added followed by PyBOP (14mg, 2.0eq.). The reaction mixture was stirred at room temperature for 15min. then a solution of 11-ddGTP (0.0083mmol, Na₂CO₃-NaHCO₃ pH 8.5) added in one portion. The reaction mixture was stirred at room temperature for 3h. then applied directly to a silica gel column. The product was eluted with iPrOH:NH₄OH:H₂O (6:3:1 v/v/v) then purified by ion exchange chromatography (as for 6) followed by C18 RP-HPLC (1.75µmol yield, 21%).

### 5.3 Sequencing Results

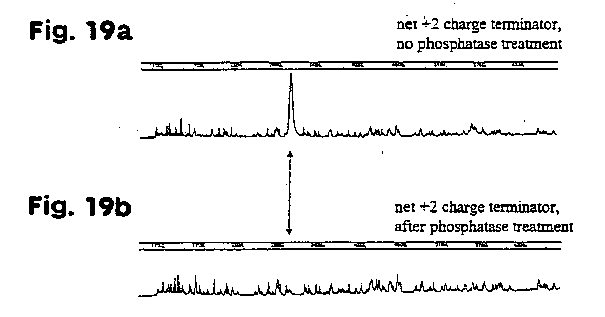

The electropherogram shown in Figure 19 was obtained when 27 was used in a sequencing reaction. The +2 charged terminator was used in a sequencing reaction and loaded directly on to a slab gel. The same experiment was repeated, however the reaction mixture was treated with phosphatase prior to loading on a gel to remove phosphates from the unincorporated dye-labeled dideoxynucleotides remaining in the reaction mixture. This leaves all terminator derived products with an overall positive charge causing them to migrate in the opposite direction as the sequence products during electrophoresis. It is clear from the electropherogram that the colored by-products are absent from the sequence when phosphatase is used to break down the terminator products.

### 6. Positively charged extended linker arms as applied to direct load

### 6.1 Chemistry

Another example of dyes attached to a positively charged linker arm is shown below;

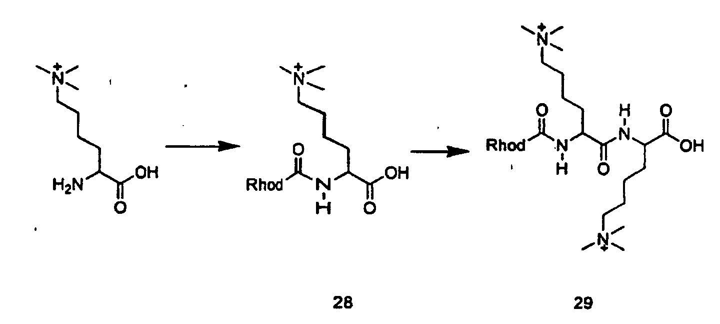

In this example, the rhodamine dye R6G is attached to ε-N,N,N-trimethyllysine which contains a formalized positive charge from the ε quaternary amine. The product (28) can be further modified to yield a +2 linker arm (29) by reaction with a further molecule of the charged amino acid. Further reaction(s) would generate the desired charged structure.

### 6.2 Experimental

### α-N-(5-carboxamidorhodamine6G)-ε-N,N,N-trimethyllysine (28)

ε-N,N,N-trimethyllysine (68mg, 30.0mmol) was dissolved in DMF (6ml) then diisopropylethylamine (0.5mt, 10eq.) added followed by R6G-NHS active ester (200mg, 1.2eq.). The reaction mixture was stirred at room temperature for 16h. then evaporated to dryness *in vacuo.* The product was isolated by C18 RP HPLC (A=0.1M TEAB, B=0.1MTEAB/50%MeCN, 0-100%B over 50 min., 100ml/min). Retention time = 44min.

### α-(α'-N-(5-carboxamidorhodamine6G)-ε'-N,N,N-trimethyllysine)-ε-N,N,N-trimethyllysine (29)

α-N-(5-carboxamidorhodamine6G)-ε-N,N,N-trimethyllysine 28 (100mg, 0.15mmol) was dissolved in DMF (5ml) then diisopropylethylamine (0.3ml, 15eq.) and TSTU (67mg, 1.5eq.) added. The reaction mixture was stirred at room temperature for 1h. then ε-N,N,N-trimethyllysine (50mg, 1.5eq.) added. The solution was stirred for a further 3h. then the reaction mixture was evaporated to dryness *in vacuo*. The product was isolated by C18 RP HPLC (A=0.1M TEAB, B=0.1MTEAB/50%MeCN, 0-100%B over 90 min., 100ml/min). Retention time = 60min.

**TABLE IV**

| *Abbreviations* | |
|---|---|
| Abbreviation | Definition |
| ddNTP | 2'-3'-dideoxynucleoside triphosphate |
| ET | Energy Transfer |
| TSTU | 2-Succinimido-1,1,3,3-tetramethyluronium tetrafluoroborate |
| PyBOP | Benzotrialzol-1-yl-oxy-tris-pyrrolidino-phosphonium hexafluorophosphate |
| DMF | N,N-dimethylformamide |
| RP HPLC | Reverse Phase High Performance Liquid Chromatography |
| Et₂O | Diethyl ether |
| DMSO | Dimethyl sulfoxide |
| TEAB | Triethylammonium bicarbonate |
| MeCN | Acetonitrile |
| iPrOH | Isopropanol |
| NH₄OH | Ammonium Hydroxide |
| BSFAM | 4',5' Bis-sulfono-5-carboxyfluorescein |
| R110 | Rhodamine 110 |
| REG or R6G | Carboxyrhodamine6G |
| TAMRA | Tertamethylrhodamine |
| ROX | Carboxy-X-rhodamine |
| DMAP | 4-dimethylaminopyridine |
| 11-ddGTP | 2',2'-dideoxyguanosine triphosphate with an 11 atom linker arm |
| NHS | N-hydroxysuccinimide |

Although various embodiments of the instant invention are described in detail above, the instant invention is not limited to such specific examples. Various modifications will be readily apparent to one of ordinary skill in the art and fall within the spirit and scope of the following appended claims.

## Claims

1. A purified recombinant thermostable DNA polymerase comprising the amino acid sequence set forth in Figure 2.

2. An isolated nucleic acid that encodes a thermostable DNA polymerase, wherein said nucleic acid consists of the nucleotide sequence corresponding to the amino acid sequence set forth in Figure 2.

3. A recombinant DNA vector that comprises the nucleic acid of Claim 2.

4. A recombinant host cell transformed with the vector of Claim 3.

5. The recombinant host cell of Claim 4 that is E. coli.

6. A method of sequencing DNA comprising the step of generating chain terminated fragments from the DNA template to be sequenced with the DNA polymerase of Claim I in the presence of at least one chain terminating agent and one or more nucleotide triphosphates, and determining the sequence of said DNA from the sizes of said fragments.

7. A method according to Claim 6, wherein the chain terminating agent comprises a labeled nucleic acid terminator having a net positive or a net. negative charge.

8. A method for synthesizing a fluorescently labeled polynucleotide, said method comprising the step of mixing a DNA polymerase according to Claim I with a primed template.

9. A method according to Claim 8, wherein the primed template is a primed template in a chain termination sequencing reaction.

10. A method according to Claim 8, wherein the primed template is a primed template in a polymerase chain reaction.

11. A kit for fluorescently labeling a polynucleotide, the kit comprising a DNA polymerase according to Claim 1 and a fluorescently labeled nucleotide.

12. A kit according to Claim 10, wherein the fluorescently labeled nucleotide comprises a nucleic acid terminator having a net negative or a net positive charge.

13. A kit for sequencing DNA comprising the DNA polymerase of Claim 1.

14. A purified recombinant thermostable DNA polymerase comprising the amino acid sequence set forth in Figure 3.

15. An isolated nucleic acid that encodes a thermostable DNA polymerase, wherein said nucleic acid consists of the nucleotide sequence corresponding to the amino acid sequence set forth in Figure 3.

16. A recombinant DNA vector that comprises the nucleic acid of Claim 15.

17. A recombinant host cell transformed with the vector of Claim 16.

18. The recombinant host cell of Claim 17 that is E. coli.

19. A method of sequencing DNA comprising the step of generating chain terminated fragments from the DNA template to be sequenced with the DNA polymerase of Claim 14 in the presence of at least one chain terminating agent and one or more nucleotide triphosphates, and determining the sequence of said DNA from the sizes of said fragments.

20. A method according to Claim 19, wherein the chain terminating agent comprises a labeled nucleic acid terminator having a net positive or a net negative charge.

21. A method for synthesizing a fluorescently labeled polynucleotide, said method comprising the step of mixing a DNA polymerase according to Claim 14 with a primed template.

22. A method according to Claim 21, wherein the primed template is a primed template in a chain termination sequencing reaction.

23. A method according to Claim 21, wherein the primed template is a primed template in a polymerase chain reaction.

24. A kit for fluorescently labeling a polynucleotide, the kit comprising a DNA polymerase according to Claim 14 and a fluorescently labeled nucleotide.

25. A kit according to Claim 24, wherein the fluorescently labeled nucleotide comprises a nucleic acid terminator having a net negative or a net positive charge.

26. A kit for sequencing DNA comprising the DNA polymerase of Claim 14.

## Patentansprüche

1. Gereinigte rekombinante thermostabile DNA-Polymerase mit der in Figur 2 dargestellten Aminosäuresequenz.

2. Isolierte Nucleinsäure, die eine thermostabile DNA-Polymerase kodiert, wobei die Nucleinsäure aus der Nucleotidsequenz entsprechend der in Figur 2 dargestellten Aminosäuresequenz besteht.

3. Rekombinanter DNA-Vektor, der die Nucleinsäure des Anspruchs 2 umfasst.

4. Rekombinante Wirtszelle, die mit dem Vektor nach Anspruch 3 transformiert ist

5. Rekombinante Wirtszelle nach Anspruch 4, bei der es sich um E. coli handelt.

6. Verfahren zum Sequenzieren von DNA, bei dem Kettenabbruchfragmente aus dem DNA-Template, das mit der DNA-Polymerase des Anspruchs 1 sequenziert werden soll, in Anwesenheit mindestens eines Kettenabbruchmittels und eines oder mehrerer Nucleotidtriphosphate erzeugt werden und die Sequenz der DNA aus den Größen der Fragmente bestimmt wird.

7. Verfahren nach Anspruch 6, bei dem das Kettenabbruchmittel einen markierten Nucleinsäureterminator mit einer positiven oder negativen Nettoladung umfasst.

8. Verfahren zum Synthetisieren eines fluoreszenzmarkierten Polynucleotids, wobei das Verfahren den Schritt des Mischens einer DNA-Polymerase nach Anspruch 1 mit einem Template, an das ein Primer angelagert ist, umfasst.

9. Verfahren nach Anspruch 8, bei dem das Template, an das ein Primer angelagert ist, ein Template, an das ein Primer angelagert ist, in einer Kettenabbruch-Sequenzierungsreaktion ist.

10. Verfahren nach Anspruch 8, bei dem das Template, an das ein Primer angelagert ist, ein Template, an das ein Primer angelagert ist, in einer Polymerase-Kettenreaktion ist.

11. Kit zum Fluoreszenzmarkieren eines Polynucleotids, wobei das Kit eine DNA-Polymerase nach Anspruch 1 und ein fluoreszenzmarkiertes Nucleotid umfasst.

12. Kit nach Anspruch 11, wobei das fluoreszenzmarkierte Nucleotid einen Nueleinsäureterminator mit einer positiven oder negativen Nettoladung umfasst.

13. Kit zum Sequenzieren von DNA mit der DNA-Polymerase nach Anspruch 1.

14. Gereinigte rekombinante thermostabile DNA-Polymerase mit der in Figur 3 dargestellten Aminosäuresequenz.

15. Isolierte Nucleinsäure, die eine thermostabile DNA-Polymerase kodiert, wobei die Nucleinsäure aus der Nucleotidsequenz entsprechend der in Figur 3 dargestellten Aminosäuresequenz besteht.

16. Rekombinanter DNA-Vektor, der die Nucleinsäure des Anspruchs 15 umfasst.

17. Rekombinante Wirtszelle, die mit dem Vektor nach Anspruch 16 transformiert ist.

18. Rekombinante Wirtszelle nach Anspruch 17, bei der es sich um E. coli handelt.

19. Verfahren zum Sequenzieren von DNA, bei dem Kettenabbruchfragmente aus dem DNA-Template, das mit der DNA-Polymerase des Anspruchs 14 sequenziert werden soll, in Anwesenheit mindestens eines Kettenabbruchmittels und eines oder mehrerer Nucleotidtriphosphate erzeugt werden und die Sequenz der DNA aus den Größen der Fragmente bestimmt wird.

20. Verfahren nach Anspruch 19, bei dem das Kettenabbruchmittel einen markierten Nucleinsäureterminator mit einer positiven oder negativen Nettoladung umfasst.

21. Verfahren zum Synthetisieren eines fluoreszenzmarkierten Polynucleotids, wobei das Verfahren den Schritt des Mischens einer DNA-Polymerase nach Anspruch 14 mit einem Template, an das ein Primer angelagert ist, umfasst.

22. Verfahren nach Anspruch 21, bei dem das Template, an das ein Primer angelagert ist, ein Template, an das ein Primer angelagert ist, in einer Kettenabbruch-Sequenzierungsreaktion ist.

23. Verfahren nach Anspruch 21, bei dem das Template, an das ein Primer angelagert ist, ein Template, an das ein Primer angelagert ist, in einer Polymerase-Kettenreaktion ist.

24. Kit zum Fluoreszenzmarkieren eines Polynucleotids, wobei das Kit eine DNA-Polymerase nach Anspruch 14 und ein fluoreszenzmarkiertes Nucleotid umfasst.

25. Kit nach Anspruch 24, bei dem das fluoreszenzmarkierte Nucleotid einen Nucleinsäureterminator mit einer positiven oder negativen Nettoladung umfasst.

26. Kit zum Sequenzieren von DNA mit der DNA-Polymerase nach Anspruch 14.

## Revendications

1. ADN polymérase recombinante purifiée thermiquement stable comprenant la séquence d'acides aminés montrée sur la figure 2.

2. Acide nucléique isolé qui code une ADN polymérase thermiquement stable, dans lequel ledit acide nucléique est formé par la séquence de nucléotides correspondant à la séquence d'acides aminés montrée sur la figure 2.

3. Vecteur d'ADN recombinant qui comprend l'acide nucléique selon la revendication 2.

4. Cellule hôte recombinante transformée avec le vecteur selon la revendication 3.

5. Cellule hôte recombinante selon la revendication 4 qui est E. Coli.

6. Procédé de séquençage d'ADN comprenant l'étape de production de fragments de terminaison en chaîne à partir de la matrice d'ADN pour être séquencé avec l'ADN polymérase selon la revendication 1 en présence d'au moins un agent de terminaison de chaîne et d'un ou plusieurs nucléotides triphosphates, et de détermination de la séquence dudit ADN à partir des tailles desdits fragments.

7. Procédé selon la revendication 6, dans lequel l'agent de terminaison de chaîne comprend un élément de terminaison d'acide nucléique marqué présentant une charge positive nette ou une charge négative nette.

8. Procédé de synthèse d'un polynucléotide marqué de manière fluorescente, ledit procédé comprenant l'étape de mélange d'une ADN polymérase selon la revendication 1 avec une matrice nucléique hybridée à une amorce.

9. Procédé selon la revendication 8, dans lequel la matrice nucléique hybridée à une amorce est une matrice nucléique hybridée à une amorce dans une réaction de séquençage de terminaison en chaîne.

10. Procédé selon la revendication 8, dans lequel la matrice nucléique hybridée à une amorce est une matrice nucléique hybridée à une amorce dans une réaction de polymérase en chaîne.

11. Nécessaire de marquage de manière fluorescente d'un polynucléotide, le nécessaire comprenant une ADN polymérase selon la revendication 1 et un nucléotide marqué de manière fluorescente.

12. Nécessaire selon la revendication 11, dans lequel le nucléotide marqué de manière fluorescente comprend un élément de terminaison d'acide nucléique présentant une charge négative nette ou une charge positive nette.

13. Nécessaire de séquençage d'ADN comprenant la ADN polymérase selon la revendication 1.

14. ADN polymérase recombinante purifiée thermiquement stable, comprenant la séquence d'acides aminés montrée sur la figure 3.

15. Acide nucléique isolé qui code une ADN polymérase thermiquement stable, dans lequel ledit acide nucléique est formé par la séquence de nucléotides correspondant à la séquence d'acides aminés montrée sur la figure 3.

16. Vecteur ADN recombinant qui comprend l'acide nucléique selon la revendication 15.

17. Cellule hôte recombinante transformée avec le vecteur selon la revendication 16.

18. Cellule hôte recombinante selon la revendication 17 qui est E.Coli.

19. Procédé de séquençage d'ADN comprenant l'étape de production de fragments de terminaison en chaîne à partir de la matrice d'ADN pour être séquencé avec l'ADN polymérase selon la revendication 14 en présence d'au moins un agent de terminaison de chaîne et d'un ou plusieurs nucléotides triphosphates, et la détermination de la séquence dudit ADN à partir des tailles desdits fragments.

20. Procédé selon la revendication 19, dans lequel l'agent de terminaison de chaîne comprend un élément de terminaison d'acide nucléique marqué présentant une charge positive nette ou une charge négative nette.

21. Procédé de synthèse d'un polynucléotide marqué de manière fluorescente, ledit procédé comprenant l'étape de mélange d'une ADN polymérase selon la revendication 14 avec une matrice nucléique hybridée à une amorce.

22. Procédé selon la revendication 21, dans lequel la matrice nucléique hybridée à une amorce est une matrice nucléique hybridée à une amorce dans une réaction de séquençage de terminaison en chaîne.

23. Procédé selon la revendication 21, dans lequel la matrice nucléique hybridée à une amorce est une matrice nucléique hybridée à une amorce dans une réaction de polymérase en chaîne.

24. Nécessaire destiné à marquer de manière fluorescente un polynucléotide, le nécessaire comprenant une ADN polymérase selon la revendication 14 et un nucléotide marqué de manière fluorescente.

25. Nécessaire selon la revendication 24, dans lequel le nucléotide marqué de manière fluorescente comprend un élément de terminaison d'acide nucléique présentant une charge négative nette ou une charge positive nette.

26. Nécessaire de séquençage d'ADN comprenant la polymérase ADN selon la revendication 14.


### SEQUENCE LISTING

<110> Davis, Maria
   Nelson, John
   Kumar, shiv
   Finn, Patrick J.
   Nampalli, Satyaam
   Flicke, Parke
<120> TaQ DNA Polymerase Having an Amino Acid Substitution at E681 and Homologs Thereof Exhibiting Improved Salt Tolerance
<130> PB9944
<140> PCT/US00/22150
   <141> 2000-08-10
<150> 60/150,167
   <151> 1999-08-21
<150> 60/154,739
   <151> 1999-09-17
<160> 3
<170> PatentIn Ver. 2.1
<210> 1
   <211> 832
   <212> PRT
   <213> Thermus aquaticus
<400> 1
<210> 2
   <211> 560
   <212> PRT
   <213> Thermus aquaticus
<400> 2
   Met Leu Glu Arg Leu Glu Phe Gly Ser Leu Leu His Glu Phe Gly Leu
   1 5 10 15
   Leu Glu Ser Pro Lys Ala Leu Glu Glu Ala Pro Trp Pro Pro Pro Glu
   20 25 30
   Gly Ala Phe Val Gly Phe Val Leu Ser Arg Lys Glu Pro Met Trp Ala
   35 40 45
   Asp Leu Leu Ala Leu Ala Ala Ala Arg Gly Gly Arg Val His Arg Ala
   50 55 60
   Pro Glu Pro Tyr Lys Ala Leu Arg Asp Leu Lys Glu Ala Arg Gly Leu
   65 70 75 80
   Leu Ala Lys Asp Leu Ser Val Leu Ala Leu Arg Glu Gly Leu Gly Leu
   85 90 95
   Pro Pro Gly Asp Asp Pro Met Leu Leu Ala Tyr Leu Leu Asp Pro Ser
   100 105 110
   Asn Thr Thr Pro Glu Gly Val Ala Arg Arg Tyr Gly Gly Glu Trp Thr
   115 120 125
   Glu Glu Ala Gly Glu Arg Ala Ala Leu Ser Glu Arg Leu Phe Ala Asn
   130 135 140
   Leu Trp Gly Arg Leu Glu Gly Glu Glu Arg Leu Leu Trp Leu Tyr Arg
   145 150 155 160
   Glu Val Glu Arg Pro Leu Ser Ala Val Leu Ala His Met Glu Ala Thr
   165 170 175
   Gly Val Arg Leu Asp Val Ala Tyr Leu Arg Ala Leu Ser Leu Glu Val
   180 185 190
   Ala Glu Glu Ile Ala Arg Leu Glu Ala Glu Val Phe Arg Leu Ala Gly
   195 200 205
   His Pro Phe Asn Leu Asn Ser Arg Asp Gln Leu Glu Arg Val Leu Phe
   210 215 220
   Asp Glu Leu Gly Leu Pro Ala Ile Gly Lys Thr Glu Lys Thr Gly Lys
   225 230 235 240
   Arg Ser Thr Ser Ala Ala Val Leu Glu Ala Leu Arg Glu Ala His Pro
   245 250 255
   Ile Val Glu Lys Ile Leu Gln Tyr Arg Glu Leu Thr Lys Leu Lys Ser
   260 265 270
   Thr Tyr Ile Asp Pro Leu Pro Asp Leu Ile His Pro Arg Thr Gly Arg
   275 280 285
   Leu His Thr Arg Phe Asn Gln Thr Ala Thr Ala Thr Gly Arg Leu Ser
   290 295 300
   Ser Ser Asp Pro Asn Leu Gln Asn Ile Pro Val Arg Thr Pro Leu Gly
   305 310 315 320
   Gln Arg Ile Arg Arg Ala Phe Ile Ala Glu Glu Gly Trp Leu Leu Val
   325 330 335
   Ala Leu Asp Tyr Ser Gln Ile Glu Leu Arg Val Leu Ala His Leu Ser
   340 345 350
   Gly Asp Glu Asn Leu Ile Arg Val Phe Gln Glu Gly Arg Asp Ile His
   355 360 365
   Thr Glu Thr Ala Ser Trp Met Phe Gly Val Pro Arg Glu Ala Val Asp
   370 375 380
   Pro Leu Met Arg Arg Ala Ala Lys Thr Ile Asn Tyr Gly Val Leu Tyr
   385 390 395 400
   Gly Met Ser Ala His Arg Leu Ser Gln Arg Leu Ala Ile Pro Tyr Glu
   405 410 415
   Glu Ala Gln Ala Phe Ile Glu Arg Tyr Phe Gln Ser Phe Pro Lys Val
   420 425 430
   Arg Ala Trp Ile Glu Lys Thr Leu Glu Glu Gly Arg Arg Arg Gly Tyr
   435 440 445
   Val Glu Thr Leu Phe Gly Arg Arg Arg Tyr Val Pro Asp Leu Glu Ala
   450 455 460
   Arg Val Lys Ser Val Glu Ala Ala Glu Arg Met Ala Phe Asn Met Pro
   465 470 475 480
   Val Gln Gly Thr Ala Ala Asp Leu Met Lys Leu Ala Met Val Lys Leu
   485 490 495
   Phe Pro Arg Leu Glu Glu Met Gly Ala Arg Met Leu Leu Gln Val His
   500 505 510
   Asp Glu Leu Val Leu Glu Ala Pro Lys Glu Arg Ala Glu Ala Val Ala
   515 520 525
   Arg Leu Ala Lys Glu Val Met Glu Gly Val Tyr Pro Leu Ala Val Pro
   530 535 540
   Leu Glu Val Glu Val Gly Ile Gly Glu Asp Trp Leu Ser Ala Lys Glu
   545 550 555 560
<210> 3
   <211> 830
   <212> PRT
   <213> Thermus aquaticus
<400> 3
   Met Arg Gly Met Leu Pro Leu Phe Glu Pro Lys Gly Arg Val Leu Leu
   1               5                   10                  15
   Val Ala Gly His His Leu Ala Tyr Arg Thr Phe His Ala Leu Lys Gly
               20                  25                  30
   Leu Thr Thr Ser Arg Gly Glu Pro Val Gln Ala Val Tyr Gly Phe Ala
           35                  40                  45
   Lys Ser Leu Leu Lys Ala Leu Lys Glu Asp Gly Asp Ala Val Ile Val
       50                  55                  60
   Val Phe Asp Ala Lys Ala Pro Ser Phe Arg His Glu Ala Tyr Gly Gly
   65                  70                  75                  80
   Tyr Lys Ala Gly Arg Ala Pro Thr Pro Glu Asp Phe Pro Arg Gln Leu
                   85                  90                  95
   Ala Leu Ile Lys Glu Leu Val Asp Leu Leu Gly Leu Ala Arg Leu Glu
               100                 105                 110
   Val Pro Gly Tyr Glu Ala Asp Asp Val Leu Ala Ser Leu Ala Lys Lys
           115                 120                 125
   Ala Glu Lys Glu Gly Tyr Glu Val Arg Ile Leu Thr Ala Asp Lys Asp
       130                 135                 140
   Leu Tyr Gln Leu Leu Ser Asp Arg Ile His Val Leu His Pro Glu Gly
   145                 150                 155                 160
   Tyr Leu Ile Thr Pro Ala Trp Leu Trp Glu Lys Tyr Gly Leu Arg Pro
                   165                 170                 175
   Asp Gln Trp Ala Asp Tyr Arg Ala Leu Thr Gly Asp Glu Ser Asp Asn
               180                 185                 190
   Leu Pro Gly Val Lys Gly Ile Gly Glu Lys Thr Ala Arg Lys Leu Leu
           195                 200                 205
   Glu Glu Trp Gly Ser Leu Glu Ala Leu Leu Lys Asn Leu Asp Arg Leu
       210                 215                 220
   Lys Pro Ala Ile Arg Glu Lys Ile Leu Ala His Met Asp Asp Leu Lys
   225                 230                 235                 240
   Leu Ser Trp Asp Leu Ala Lys Val Arg Thr Asp Leu Pro Leu Glu Val
                   245                 250                 255
   Asp Phe Ala Lys Arg Arg Glu Pro Asp Arg Glu Arg Leu Arg Ala Phe
               260                 265                 270
   Leu Glu Arg Leu Glu Phe Gly Ser Leu Leu His Glu Phe Gly Leu Leu
   275 280 285
   Glu Ser Pro Lys Ala Leu Glu Glu Ala Pro Trp Pro Pro Pro Glu Gly
   290 295 300
   Ala Phe Val Gly Phe Val Leu Ser Arg Lys Glu Pro Met Trp Ala Asp
   305 310 315 320
   Leu Leu Ala Leu Ala Ala Ala Arg Gly Gly Arg Val His Arg Ala Pro
   325 330 335
   Tyr Lys Ala Leu Arg Asp Leu Lys Glu Ala Arg Gly Leu Leu Ala Lys
   340 345 350
   Asp Leu Ser Val Leu Ala Leu Arg Glu Gly Leu Gly Leu Pro Pro Gly
   355 360 365
   Asp Asp Pro Met Leu Leu Ala Tyr Leu Leu Asp Pro Ser Asn Thr Thr
   370 375 380
   Pro Glu Gly Val Ala Arg Arg Tyr Gly Gly Glu Trp Thr Glu Glu Ala
   385 390 395 400
   Gly Glu Arg Ala Ala Leu Ser Glu Arg Leu Phe Ala Asn Leu Trp Gly
   405 410 415
   Arg Leu Glu Gly Glu Glu Arg Leu Leu Trp Leu Tyr Arg Glu Val Glu
   420 425 430
   Arg Pro Leu Ser Ala Val Leu Ala His Met Glu Ala Thr Gly Val Arg
   435 440 445
   Leu Asp Val Ala Tyr Leu Arg Ala Leu Ser Leu Glu Val Ala Glu Glu
   450 455 460
   Ile Ala Arg Leu Glu Ala Glu Val Phe Arg Leu Ala Gly His Pro Phe
   465 470 475 480
   Asn Leu Asn Ser Arg Asp Gln Leu Glu Arg Val Leu Phe Asp Glu Leu
   485 490 495
   Gly Leu Pro Ala Ile Gly Lys Thr Glu Lys Thr Gly Lys Arg Ser Thr
   500 505 510
   Ser Ala Ala Val Leu Glu Ala Leu Arg Glu Ala His Pro Ile Val Glu
   515 520 525
   Lys Ile Leu Gln Tyr Arg Glu Leu Thr Lys Leu Lys Ser Thr Tyr Ile
   530 535 540
   Asp Pro Leu Pro Asp Leu Ile His Pro Arg Thr Gly Arg Leu His Thr
   545 550 555 560
   Arg Phe Asn Gln Thr Ala Thr Ala Thr Gly Arg Leu Ser Ser Ser Asp
   565 570 575
   Pro Asn Leu Gln Asn Ile Pro Val Arg Thr Pro Leu Gly Gln Arg Ile
   580 585 590
   Arg Arg Ala Phe Ile Ala Glu Glu Gly Trp Leu Leu Val Ala Leu Asp
           595                     600                     605
   Tyr Ser Gln Ile Glu Leu Arg Val Leu Ala His Leu Ser Gly Asp Glu
       610                     615                     620
   Asn Leu Ile Arg Val Phe Gln Glu Gly Arg Asp Ile His Thr Glu Thr
   625                     630                     635                 640
   Ala Ser Trp Met Phe Gly Val Pro Arg Glu Ala Val Asp Pro Leu Met
                   645                     650                     655
   Arg Arg Ala Ala Lys Thr Ile Asn Tyr Gly Val Leu Tyr Gly Met Ser
               660                     665                     670
   Ala His Arg Leu Ser Gln Arg Leu Ala Ile Pro Tyr Glu Glu Ala Gln
           675                     680                     685
   Ala Phe Ile Glu Arg Tyr Phe Gln Ser Phe Pro Lys Val Arg Ala Trp
       690                     695                     700
   Ile Glu Lys Thr Leu Glu Glu Gly Arg Arg Arg Gly Tyr Val Glu Thr
   705                     710                     715                 720
   Leu Phe Gly Arg Arg Arg Tyr Val Pro Asp Leu Glu Ala Arg Val Lys
                   725                     730                     735
   Ser Val Arg Glu Ala Ala Glu Arg Met Ala Phe Asn Met Pro Val Gln
               740                     745                     750
   Gly Thr Ala Ala Asp Leu Met Lys Leu Ala Met Val Lys Leu Phe Pro
           755                     760                     765
   Arg Leu Glu Glu Met Gly Ala Arg Met Leu Leu Gln Val His Asp Glu
       770                     775                     780
   Leu Val Leu Glu Ala Pro Lys Glu Arg Ala Glu Ala Val Ala Arg Leu
   785                     790                     795                 800
   Ala Lys Glu Val Met Glu Gly Val Tyr Pro Leu Ala Val Pro Leu Glu
                   805                     810                     815
   Val Glu Val Gly Ile Gly Glu Asp Trp Leu Ser Ala Lys Glu
               820                     825                     830